(19) Europäisches Patentamt
European Patent Office
Office européen des brevets

(11) Veröffentlichungsnummer: **0 000 395**
**B1**

(12) # EUROPÄISCHE PATENTSCHRIFT

(51) Int. Cl.³: C 07 D 295/08, C 07 D 401/04, C 07 D 403/04, C 07 D 417/04, A 61 K 31/495, A 61 K 31/505

(21) Anmeldenummer: 78100365.2

(22) Anmeldetag: 11.07.78

(54) **2-Piperazinotetraline, ihre Herstellung und Verwendung als Arzneimittel**

(30) Priorität: 18.07.77 CH 8863/77
08.11.77 CH 13583/77

(43) Veröffentlichungstag der Anmeldung:
24.01.79 Patentblatt 79/02

(45) Bekanntmachung des Hinweises auf die Patenterteilung:
07.01.81 Patentblatt 81/01

(84) Benannte Vertragsstaaten:
BE CH DE FR GB LU NL SE

(56) Entgegenhaltungen:
DE-B-1 190 466
FR-A-2 100 935
FR-A-2 323 388

(73) Patentinhaber: SANDOZ AG
Lichtstrasse 35
CH-4002 Basel (CH)

(72) Erfinder: Seiler, Max-Peter, Dr.
Clarastrasse 21
CH-4058 Basel (CH)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 395 B1

2-Piperazinotetraline, ihre Herstellung und Verwendung als Arzneimittel

Die Erfindung betrifft neue 2-Piperazinotetraline.

Aus der deutschen Auslegeschrift 1.190.466 sind schon 2-Piperazinotetraline bekannt, die im Benzolring des Tetralingerüsts unsubstituiert sind. Weiter sind in der französischen Patentschrift 2.100.935 Derivate des 2-Piperazino-dihydronaphthalins beschrieben.

Gegenstand der vorliegenden Erfindung sind neue 2-Piperazinotetraline der Formel I

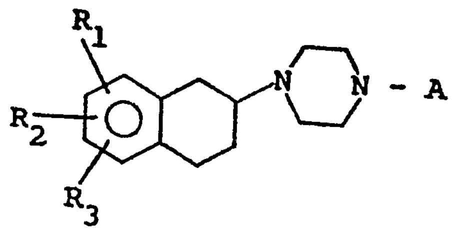

I

worin

$R_1$ Hydroxy, Alkoxy mit 1—4 C-Atomen, Alkanoyloxy mit 1—20 C-Atomen oder eine

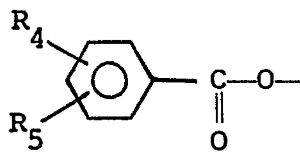
Gruppe,

$R_4$ und $R_5$ unabhängig voneinander Wasserstoff, Fluor, Chlor, Brom, Jod, Alkyl mit 1—4 C-Atomen oder Alkoxy mit 1—4 C-Atomen oder

$R^4$ und $R^5$ an benachbarten C-Atomen zusammen eine Methylendioxygruppe,

$R_2$ und $R_3$ unabhängig voneinander Wasserstoff,

Hydroxy, Alkoxy mit 1—4 C-Atomen, Alkanoyloxy mit 1—20 C-Atomen oder eine

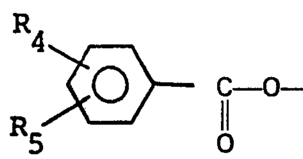
Gruppe oder

$R_1$ und $R_2$ an benachbarten C-Atomen zusammen eine Methylendioxygruppe,

A a) eine 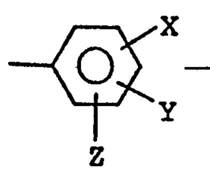 —Gruppe

X, Y und Z unabhängig voneinander Wasserstoff, Hydroxy, Alkyl mit 1—4 C-Atomen, Alkoxy mit 1—4 C-Atomen, Alkanoyloxy mit 1—20 C-Atomen,

eine 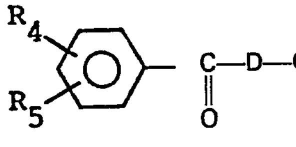 Gruppe, Fluor, Chlor,

Brom, Jod, $CF_3$, SH, Alkylthio mit 1—4 C-Atomen oder Alkanoylthio mit 1—20 C-Atomen oder X und Y an benachbarten C-Atomen zusammen eine Methylendioxygruppe,

D O oder S

b) eine 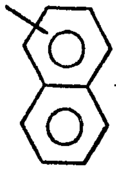 —Gruppe

c) einen Fünf- oder Sechsring der Formel

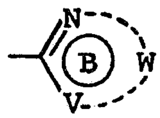

worin
V entweder zweiwertig ist und für O, S, NH oder $CH_2$ steht oder dreiwertig ist und für N oder CH steht,
W für eine gesättigte oder ungesättigte Alkylenkette mit 2 oder 3 C-Atomen steht
under der Ring Ⓑ eine, zwei oder drei Doppelbindungen enthalten kann,

bedeuten, sowie ihre Säureadditionssalze und Verfahren zu ihrer Herstellung.

Die Verbindungen der Formel I können in Form von Enantiomeren oder in Form von Racematen auftreten.

In der obigen Formel können alle Alkyl-, Alkoxy- und Alkanoylgruppen geradkettig oder verzweigt sein.

$R_1$, $R_2$ und $R_3$ stehen als Alkoxygruppen bevorzugt für die Methoxygruppe.

$R_1$ steht vorzugsweise für die freie OH-Gruppe oder für eine Alkoxygruppe, besonders für die freie OH-Gruppe.

Der Rest $R_1$ befindet sich vorzugsweise in Stellung 6 des Tetralinringes.

$R_2$ und $R_3$ bedeuten vorzugsweise Wasserstoff oder die freie OH-Gruppe, besonders Wasserstoff.

X, Y und Z stehen als Alkylreste bevorzugt für die Methylgruppe, als Alkoxygruppe für die Methoxygruppe.

Der Rest X steht vorzugsweise für eine Alkoxy- oder eine Alkylgruppe, die Reste Y und Z für Wasserstoff, Hydroxy- oder Alkoxygruppen.

X steht bevorzugt in Stellung 2 des Phenylrestes.

A hat vorzugsweise die unter a) angegebenen Bedeutungen.

Die Substituenten $R_4$ und $R_5$ können in den Resten $R_1$, $R_2$, $R_3$, X, Y und Z unabhängig voneinander die obenerwähnten Bedeutungen haben.

Erfindungsgemäss gelangt man

a) zu den Verbindungen der Formel Ia und ihren Säureadditionssalzen

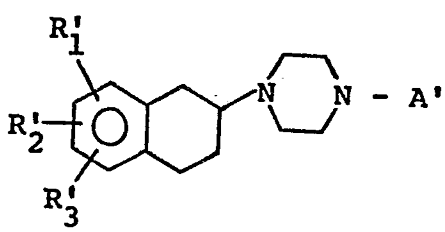

Ia

worin
$R_1'$ für eine Alkoxygruppe mit 1—4 C-Atomen und
$R_2'$ und $R_3'$ unabhängig voneinander für Wasserstoff oder eine Alkoxygruppe mit 1—4 C-Atomen oder
$R_1'$ und $R_2'$ zusammen für eine —O—$CH_2$—O—Gruppe stehen und
A' die gleichen Bedeutungen wie A, mit Ausnahme des durch mindestens eine Alkanoyloxy-, Alkanoylthio- oder 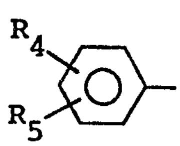 COD—Gruppe

substituierten Phenylrestes, hat, indem man Verbindungen der Formel II

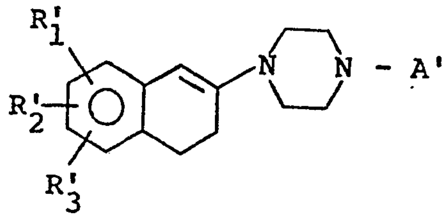

reduziert,
b) zu den Verbindungen der Formel Ib und ihren Säureadditionssalzen

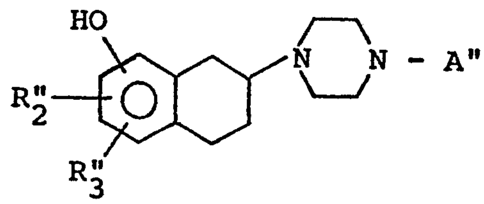

Ib

worin
$R_2''$ und $R_3''$ unabhängig voneinander für Wasserstoff oder Hydroxy und

A″ für eine 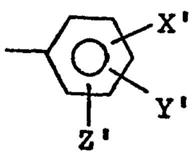 Gruppe,

worin
X′, Y′ und Z′ unabhängig voneinander Wasserstoff, Hydroxy, Alkyl mit 1—4 C-Atomen, Fluor, Chlor, Brom, Jod, $CF_3$ oder SH bedeuten,
oder für eine der oben unter b) und c) angegebenen Bedeutungen für A stehen, indem man Verbindungen der Formel Ia einer Aetherspaltung unterwirft,
c) zu den Verbindungen der Formel Ic und ihren Säureadditionssalzen

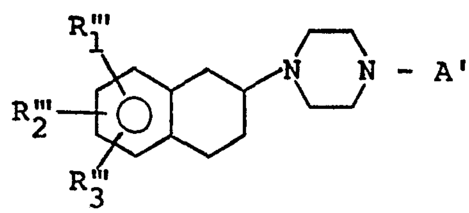

Ic

worin
$R_1'''$ für Hydroxy oder Alkoxy mit 1—4 C-Atomen,
$R_2'''$ und $R_3'''$ unabhängig voneinander für Wasserstoff, Hydroxy oder Alkoxy mit 1—4 C-Atomen stehen, indem man Verbindungen der Formel III

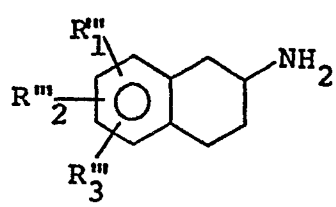

III

mit Verbindungen der Formel IV

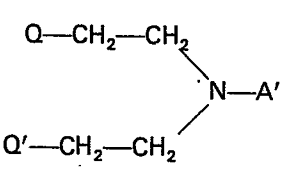

IV.

worin

Q und Q' für den Säurerest eines reaktiven Esters stehen, kondensiert,

d) zu den Verbindungen der Formel I, worin mindestens einer der Substituenten $R_1$, $R_2$, $R_3$, X, Y oder Z für eine Alkanoyloxy- oder

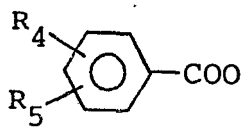

— Gruppe und/oder einer der Substituenten X, Y oder Z für eine Alkanoylthio- oder

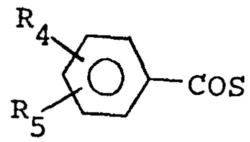

Gruppe steht, und ihren Säureadditionssalzen, indem man Verbindungen der Formel I, worin mindestens einer der Substituenten $R_1$, $R_2$, $R_3$, X, Y oder Z für eine freie OH-Gruppe und/oder einer der Substituenten X, Y oder Z für eine freie SH-Gruppe steht, mit einem reaktiven Derivat einer Alkancarbonsäure mit 1—20 C-Atomen oder einer aromatischen Carbonsäure der Formel

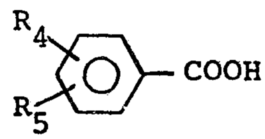

acyliert und die erhaltenen Verbindungen der Formel I gegebenenfalls in ihre Säureadditionssalze überführt.

Die erfindungsgemäss erhaltenen Verbindungen der Formel I können in Form der freien Basen oder ihrer Säureadditionssalze vorliegen. Die freien Basen können auf an sich bekannte Weise in ihre Säureadditionssalze überführt werden und umgekehrt. So können die erfindungsgemässen Verbindungen der Formel I z.B. mit anorganischen Säuren wie Chlorwasserstoffsäure oder mit organischen Säuren wie Maleinsäure Säureadditionssalze bilden.

Das Verfahren a) kann in an sich bekannter Weise durchgeführt werden, z.B. durch katalytische Hydrierung. Geeignete Katalysatoren umfassen Palladium-Kohle, Platin oder Raney-Nickel vorzugsweise Palladium-Kohle. Zweckmässigerweise wird ein inertes organisches Lösungsmittel, z.B. Aethanol oder Dimethylformamid, verwendet. Die Reduktion kann auch mit komplexen Metallhydriden, z.B. Natriumborhydrid, in einem organischen Lösungsmittel wie Trifluoressigsäure durchgeführt werden. Das Verfahren wird zweckmässigerweise bei Temperaturen zwischen 10 und 50°C, vorzugsweise 20 und 30°C, durchgeführt.

Das Verfahren b) kann in einer für die Spaltung von Aethern bekannten Weise durchgeführt werden. Die Spaltung erfolgt zweckmässigerweise durch Einwirkung von abspaltenden Agentien, beispielsweise Jodwasserstoffsäure, Bromwasserstoffsäure oder Chlorwasserstoffsäure, vorzugsweise in Wasser oder Essigsäure, zweckmässigerweise bei Temperaturen von 0 bis 100°, oder Bortribromid, vorzugsweise in Methylenchlorid, zweckmässigerweise von 0 bis 50°C. Bei Verwendung von Chlorwasserstoff arbeitet man vorzugsweise bei einem Druck von 1 bis 10 Atm. Falls X, Y oder Z für Alkoxygruppen in den Stellungen 2 und 6 des Phenylringes stehen, bleiben diese bei Verwendung von Halogenwasserstoffsäuren grösstenteils erhalten und werden nicht zu freien OH-Gruppen umgesetzt.

Die Kondensation c) kann nach an sich für die Herstellung des Piperazinringes bekannten Methoden erfolgen. Vorzugsweise werden die Verbindungen III und IV in einem inerten Lösungsmittel bei Temperaturen zwischen 60 und 120° erhitzt. Als Lösungsmittel verwendet man vorteilhafterweise Aethanol, Dimethylformamid oder höhere Alkohole. Die Kondensation kann in Gegenwart eine Base, z.B. tert. Amin oder Alkalicarbonat, durchgeführt werden. Die Reste Q und Q' stehen vorzugsweise für Chlor, Brom, Jod, eine Alkylsulfonyloxy- oder Arylsulfonyloxygruppe.

Das Verfahren d) kann in einer für die Acylierung von Phenolen bekannten Weise durchgeführt werden. Als reaktive Derivate der Carbonsäuren können beispielsweise Säurehalogenide oder Säureanhydride verwendet werden.

Die Verbindungen der Formel I können in an sich bekannter Weise isoliert und gereinigt werden.

Die optisch aktiven Verbindungen der Formel I können z.B. ausgehend von optisch aktiven Ausgangsprodukten (hergestellt nach an sich für die Spaltung von Racematen üblichen Methoden) erhalten werden.

Zu den Verbindungen der Formel II kann man gelangen, indem man beispielsweise Verbindungen der Formel V

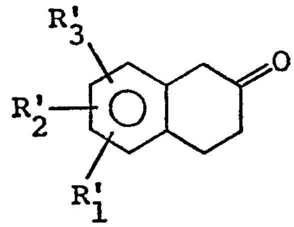

V

mit Verbindungen der Formel VI

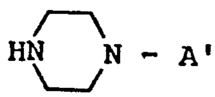

VI

umsetzt.

Die Umsetzung kann in an sich bekannter Weise durchgeführt werden. Beispielsweise kann die Umsetzung in einem inerten Lösungsmittel, z.B. Toluol, in Gegenwart katalytischer Mengen von p-Toluolsulfonsäure unter Wasserabscheidung, zweckmässigerweise bei Rückflusstemperatur des verwendeten Lösungsmittels oder in Gegenwart katalytischer Mengen einer Lewis-Säure, z.B. Titantetrachlorid, zweckmässigerweise zwischen 20 und 100°, erfolgen.

Die so erhalten Verbindungen der Formel II können ohne Isolierung direkt zu Verbindungen der Formel Ia der Reduktion in situ unterworfen werden.

Die Umsetzung von Verbindungen der Formel V mit Verbindungen der Formel VI kann jedoch direkt zu Verbindungen der Formel Ia führen, falls man die Umsetzung unter reduzierenden Bedingungen durchführt. Als Reduktionsmittel kann z.B. Wasserstoff in Gegenwart von Katalysatoren wie Raney-Nickel, Platin oder Palladium-Kohle verwendet werden. Das Verfahren wird zweckmässigerweise in einem Lösungsmittel, z.B. Essigsäure, bei Zimmertemperatur durchgeführt.

Die Verbindungen der Formeln III, IV, V und VI sind entweder bekannt oder können in an sich bekannter Weise hergestellt werden.

Die Verbindungen der Formel I zeichnen sich durch interessante pharmakodynamische Eigenschaften aus.

Sie zeigen am narkotisierten Hund eine Blutdrucksenkung und Steigerung der Durchblutung in der Arteria mesenterica, charakteristisch für Dopaminrezeptorenstimulation. Die Verbindungen der Formel I können daher zur Behandlung der Hypertonie verwendet werden. Die zu verwendenden Dosen variien naturgemäss je nach Art der Substanz, der Administration und des zu behandelnden Zustandes. Bei grösseren Säugetieren ist eine täglich zu verabreichende Menge zwischen 15 und 1000 mg angezeigt. Diese Dosis kann auch in kleineren Dosen 2—4 mal täglich oder in Retardform verabreicht werden. Eine Einheitsdosis, beispielsweise eine zur oralen Verabreichung geeignete Tablette, kann zwischen 4 und 500 mg des Wirkstoffes zusammen mit geeigneten pharmazeutisch indifferenten Hilfsstoffen enthalten.

Die Erfindung betrifft auch Heilmittel, die eine Verbindung der Formel I enthalten. Diese Heilmittel, beispielsweise eine Lösung oder eine Tablette, können nach bekannten Methoden, unter Verwendung der üblichen Hilfs- und Trägerstoffe, hergestellt werden.

In einer Gruppe der erfindungsgemässen Verbindungen bedeuten

$R_1$ Hydroxy, Alkoxy mit 1—4 C-Atomen, Alkanoyloxy mit 1—4 C-Atomen oder Benzoyloxy,

$R_2$ und $R_3$ unabhängig voneinander Wasserstoff, Hydroxy, Alkoxy mit 1—4 C-Atomen, Alkanoyloxy mit 1—4 C-Atomen oder Benzoyloxy oder

$R_1$ und $R_2$ an benachbarten C-Atomen zusammen eine Methylendioxygruppe,

A eine 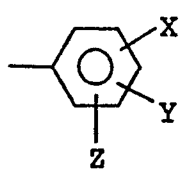 —Gruppe,

X, Y und Z unabhängig voneinander Wasserstoff, Hydroxy, Alkyl mit 1—4 C-Atomen, Alkoxy mit 1—4 C-Atomen, Alkanoyloxy mit 1—4 C-Atomen, Benzoyloxy, Fluor, Chlor oder $CF_3$ oder X und Y an benachbarten C-Atomen zusammen eine Methylendioxygruppe.

In einer zweiten Gruppe bedeuten

$R_1$ Hydroxy, Alkoxy mit 1—4 C-Atomen, Alkanoyloxy mit 1—20 C-Atomen oder eine

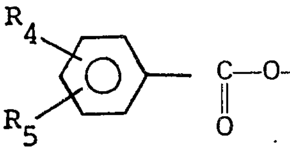—Gruppe,

$R_4$ und $R_5$ unabhängig voneinander Wasserstoff, Fluor, Chlor, Brom, Jod, Alkyl mit 1—4 C-Atomen oder Alkoxy mit 1—4 C-Atomen oder

$R^4$ und $R_5$ an benachbarten C-Atomen zusammen eine Methylendioxygruppe,

$R_2$ und $R_3$ unabhängig voneinander Wasserstoff, Hydroxy, Alkoxy mit 1—4 C-Atomen, Alkanoyloxy mit 1—20 C-Atomen oder eine

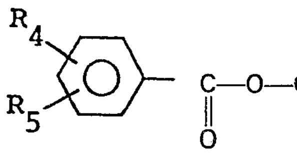—Gruppe oder

$R_1$ und $R_2$ an benachbarten C-Atomen zusammen eine Methylendioxygruppe,

A eine 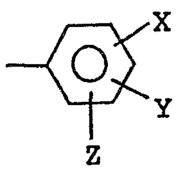—Gruppe,

X, Y und Z unabhängig voneinander Wasserstoff, Hydroxy, Alkyl mit 1—4 C-Atomen, Alkoxy mit 1—4 C-Atomen, Alkanoyloxy mit 1—20 C-Atomen, eine

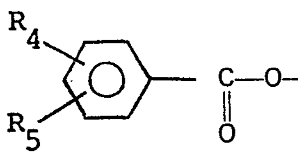—Gruppe, Fluor, Chlor, Brom, Jod,

$CF_3$, SH oder Alkylthio mit 1—4 C-Atomen oder
X und Y an benachbarten C-Atomen zusammen eine Methylendioxygruppe.

In den nachfolgenden Beispielen erfolgen alle Temperaturangaben in Celsiusgraden.

## BEISPIEL 1

1,2,3,4-Tetrahydro-6-methoxy-2-[4-(2-methylphenyl)-1-piperazinyl]-naphthalin

6 g Methoxy-2-tetralon werden zusammen mit 6 g N-(o-Tolyl)-piperazin unter Erwärmen in 100 ml Toluol gelöst, 300 mg p-Toluolsulfonsäure zugefügt und die Lösung während 24 Stunden am Rückfluss erhitzt, wobei das Wasser mit einer Dean-Stark-Falle abgetrennt wird. Das Reaktionsgemisch wird sodann zur Trockne eingedampft, das erhaltene 3,4-Dihydro-6-methoxy-2-[4-(2-methylphenyl)-1-piperazinyl]-naphthalin mit 200 ml Dimethylformamid und 300 mg 10%-igem Palladium-Kohle-Katalysator versetzt und bei Normaldruck hydriert. Nach beendeter Wasserstoffaufnahme wird der Katalysator abfiltriert, das Lösungsmittel abgedampft und der Rückstand an Kieselgel mit Methylenchlorid/Methanol (99 : 1) chromatographiert. Man erhält die Titelverbindung als Festkörper, dessen Hydrochlorid bei 286—288° schmilzt.

## BEISPIEL 2

1,2,3,4-Tetrahydro-6-methoxy-2-[4-(2-methyl-phenyl)-1-piperazinyl]-naphthalin

3,6 g 6-Methoxy-2-tetralon werden zusammen mit 3,9 g N-(o-Tolyl)-piperazin in 50 ml Essigsäure gelöst, mit 1 g 10%-igem Palladium-Kohle-Katalysator versetzt und hydriert. Nach beendeter Wasserstoffaufnahme wird der Katalysator abfiltriert, das Lösungsmittel abgedampft und der Rückstand analog Beispiel 1 an Kieselgel chromatographiert. Man erhält die Titelverbindung als Festkörper, dessen Hydrochlorid bei 286—288° schmilzt.

## BEISPIEL 3

1,2,3,4-Tetrahydro-6-hydroxy-2-[4-(2-methylphenyl)-1-piperazinyl]-naphthalin

10 g 1,2,3,4-Tetrahydro-6-methoxy-2-[4-(2-methylphenyl)-1-piperazinyl]-naphthalin werden in

200 ml 47%-iger wässriger Bromwasserstoffsäure suspendiert und unter Stickstoff während 3 Stunden am Rückfluss erhitzt. Die Bromwasserstoffsäure wird anschliessend abgedampft, das als Hydrobromid anfallende kristalline Produkt durch Extraktion mit 1n wässriger Natriumbicarbonatlösung/Methylenchlorid in die freie Base überführt und diese aus Acetonitril umkristallisiert. Die als Festkörper erhaltene Titelverbindung schmilzt bei 177—179°, deren Hydrochlorid bei 304—306°C.

BEISPIEL 4

1,2,3,4-Tetrahydro-6-hydroxy-2-[4-(2-methoxy-phenyl)-1-piperazinyl]-naphthalin

2,48 g N,N-Di(2-chloräthyl)-2-methoxyanilin werden zusammen mit 1,63 g 2-Amino-1,2,3,4-tetrahydro-6-hydroxynaphthalin in 100 ml Aethanol gelöst und anschliessend während 20 Stunden am Rückfluss gekocht. Das Lösungsmittel wird abgedampft und der Rückstand aus Methanol/Aether umkristallisiert. Man erhält die Titelverbindung in Form ihres Hydrochlorids, das bei 282—286° schmilzt.

BEISPIEL 5

1,2,3,4-Tetrahydro-6-acetoxy-2-[4-(2-methoxyphenyl)-1-piperazinyl]-naphthalin

170 mg 1,2,3,4-Tetrahydro-6-hydroxy-2-[4-(2-methoxyphenyl)-1-piperazinyl]-naphthalin werden in 2 ml Pyridin vorgelegt, 70 $\mu$l Essigsäureanhydrid unter Rühren zugetropft und die Lösung während 3 Stunden bei Zimmertemperatur stehen gelassen. Die Reaktionslösung wird anschliessend zur Trockene eingeengt, der Rückstand in Essigester aufgenommen, mit 1n Natriumcarbonatlösung gewaschen, getrocknet und zur Trockene eingedampft. Die so erhaltene rohe Titelverbindung wird anschliessend ins Hydrochlorid überführt, welches, nach Umkristallisation aus Methanol/Aether, bei 249—253° schmilzt.

BEISPIEL 6

(+- bzw. (—)-1,2,3,4-Tetrahydro-6-hydroxy-2-[4-(2-methylphenyl)-1-piperazinyl]-naphthalin

a) *(+)- bzw. (—)-2-Amino-1,2,3,4-tetrahydro-6-methoxy-naphthalin*

Zu einer Lösung von 55 g 2-Amino-1,2,3,4-tetrahydro-6-methoxy-naphthalin in 650 ml Methanol werden unter Rühren 47 g D-(—)-Mandelsäure, gelöst in 650 ml Methanol, zugetropft. Die entstandene Reaktionslösung wird während 3 Stunden bei Zimmertemperatur stehen gelassen, das ausgefallene Kristallisat abfiltriert, mit Aether gewaschen und getrocknet. Das so erhaltene (—)-Mandelsäure-Salz wird nun aus heissem Methanol umkristallisiert und der Vorgang wiederholt, bis eine Probe des jeweils durch Extraktion mit 1n Natriumcarbonatlösung/Methylenchlorid aus dem Mandelsäuresalz freigesetzten Amins einen konstanten Drehwert aufweist. Der so erhaltene freigesetzte (+)-Antipode der Titelverbindung wird ins Hydrochlorid überführt, das aus Methanol umkristallisiert, bei 262—264° schmilzt. $[\alpha]_D^{20} = +77{,}6°$ (c = 1 in Methanol)

Aus der 1. Mutterlauge des oben beschriebenen (—)-Mandelsäuresalzes wird das Amin durch Extraktion mit 1n Natriumcarbonatlösung/Methylenchlorid freigesetzt und unter Verwendung von (+)-Mandelsäure, analog dem für (—)-Mandelsäure beschriebenen Verfahren, der (—)-Antipode der Titelverbindung hergestellt, dessen Hydrochlorid, nach Umkristallisation aus Methanol, bei 263—265° schmilzt.
$[\alpha]_D^{20} = -78{,}6°$ (c = 1 in Methanol)

b) *(+)- bzw. (—)-2-Amino-1,2,3,4-tetrahydro-6-hydroxynaphthalin*

Das oben beschriebene (+)-2-Amino-1,2,3,4-tetrahydro-6-methoxynaphthalin wird durch Aetherspaltung analog Verfahren b) in die entsprechende Hydroxyverbindung überführt, in das Hydrochlorid umgewandelt und letzteres aus Methanol umkristallisiert. Man erhält so den (+)-Antipoden der Titelverbindung.
Smp. 276—278° $[\alpha]_D^{20} = +80{,}5°$ (c = 1 in Methanol)

In entsprechender Weise, unter Verwendung von (—)-2-Amino-1,2,3,4-tetrahydro-6-methoxy-naphthalin, erhält man den (—)-Antipoden der Titvelverbindung.
Smp. 276—278° $[\alpha]_D^{20} = -83{,}0°$ (c = 1 in Methanol

c) *(+)- bzw. (—)-1,2,3,4-Tetrahydro-6-hydroxy-2-[4-(2-methylphenyl)-1-piperazinyl]- naphthalin*

Ausgehend von (+)-2-Amino-1,2,3,4-tetrahydro-6-hydroxynaphthalin erhält man durch Kondensation mit N,N-Di(2-chloräthyl)-2-methylanilin, entsprechend Verfahren c), und anschliessender Umkristallisation des Produktes aus Acetonitril den (+)-Antipoden der Titelverbindung.
Smp. 147—149° $[\alpha]_D^{20} = +49{,}7°$ (c = 1 in Methanol).

Entsprechend gelangt man, ausgehend von (—)-2-Amino-1,2,3,4-tetrahydro-6-hydroxynaphthalin, zum (—)-Antipoden der Titelverbindung.
Smp. 146—148° $[\alpha]_D^{20} = -50{,}3°$ (c = 1 in Methanol)

Analog zu den Beispielen 1), 3) und 4) wurden aus den entsprechenden Ausgangsprodukten die nachfolgenden Verbindungen der Tabellen I und II hergestellt.

TABELLE I: Verbindungen der Formel

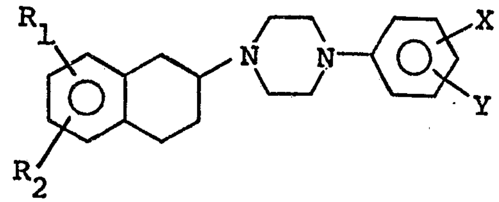

| Bsp. Nr. | $R_1$ | $R_2$ | X | Y | Analog Bsp. | Smp. | (Salzform) |
|---|---|---|---|---|---|---|---|
| 7 | 6-$CH_3O$ | 7-$CH_3O$ | 2-$CH_3$ | H | 1 | 277–280° | (Dihydrochlorid) |
| 8 | 6-OH | 7-OH | 2-$CH_3$ | H | 3 | 305–308° | (Dihydrochlorid) |
| 9 | 6-OH | H | 2-Cl | H | 4 | 303–305° | (Hydrochlorid) |
| 10 | 6-OH | H | 4-$CH_3O$ | H | 4 | 297–301° | (Dihydrochlorid) |
| 11 | 5-$CH_3O$ | H | 2-$CH_3$ | H | 4 | 281–282° | (Hydrochlorid) |
| 12 | 5-OH | H | 2-$CH_3$ | H | 3 | 333–336° | (Hydrochlorid) |
| 13 | 6-$CH_3O$ | H | 4-$CH_3O$ | H | 1 | 244–246° | (Dihydrochlorid) |
| 14 | 6-OH | H | 4-OH | H | 3 | 302–305° | (Dihydrochlorid) |
| 15 | 7-$CH_3O$ | H | 2-$CH_3$ | H | 1 | 270–271° | (Hydrochlorid) |
| 16 | 7-OH | H | 2-$CH_3$ | H | 3 | 277–279° | (Dihydrochlorid) |
| 17 | 5-$CH_3O$ | 8-$CH_3O$ | 2-$CH_3O$ | H | 1 | 291–293° | (Hydrochlorid) |
| 18 | 6-OH | H | H | H | 3 | 324–326° | (Dihydrochlorid) |
| 19 | 6-$CH_3O$ | H | 3-$CF_3$ | H | 1 | 254–255° | (Dihydrochlorid) |
| 20 | 6-OH | H | 3-$CF_3$ | H | 3 | 223–224° | (Dihydrochlorid) |

TABELLE I: Verbindungen der Formal (Fortsetzung)

| Bsp. Nr. | $R_1$ | $R_2$ | X | Y | Analog Bsp. | Smp. | (Salzform) |
|---|---|---|---|---|---|---|---|
| 21 | 6-OH | H | 3,4-O-$CH_2$–O | | 4 | 298–300° | (Dihydrochlorid) |
| 22 | 6-$CH_3$O | H | 3-$CH_3$O | 4-$CH_3$O | 4 | 238–240° | (Dihydrochlorid) |
| 23 | 6-OH | H | 3-$CH_3$O | 4-$CH_3$O | 4 | 275–278° | (Dihydrochlorid) |
| 24 | 6-OH | H | 2-$CH_3$ | 6-$CH_3$ | 3 | 306–308° | (Hydrochlorid) |
| 25 | 6-$CH_3$O | H | 4-Cl | H | 4 | 257–259° | (Dihydrochlorid) |
| 26 | 6-OH | H | 4-Cl | H | 4 | 310–312° | (Hydrochlorid) |
| 27 | 6-OH | H | 2-$C_2H_5$O | H | 4 | 304–307° | (Hydrochlorid) |
| 28 | 6-OH | H | 2-Cl | 5-$CH_3$O | 4 | 294–296° | (Hydrochlorid) |
| 29 | 6-OH | H | 2-Cl | 5-OH | 3 | 301–304° | (Hydrochlorid) |
| 30 | 6-OH | H | 2-$CH_3$O | 5-$CH_3$O | 4 | 269–271° | (Dihydrochlorid) |
| 31 | 6-OH | H | 2-$CH_3$O | 5-OH | 3 | 285–287° | (Hydrochlorid) |
| 32 | 6-OH | H | 2-$CH_3$O | 4-$CH_3$O | 4 | 282–286° | (Dihydrochlorid) |
| 33 | 6-OH | H | 2-$CH_3$O | 4-OH | 3 | 271–275° | (Dihydrochlorid) |

TABELLE II: Verbindungen der Formel

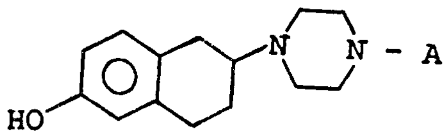

| Bsp. Nr. | A | Analog Bsp. | Smp. | (Salzform) |
|---|---|---|---|---|
| 34 | 1-Naphthyl | 3 | 302–306° | (Dihydrochlorid) |
| 35 | 2-Pyrimidyl | 4 | 270–273° | (Dihydrochlorid) |
| 36 | 2-Pyridyl | 4 | 264–266° | (Dihydrochlorid) |
| 37 | 2-Imidazolin-2-yl | 3 | | (Dihydrochlorid) |
| 38 | 2-Thiazolyl | 3 | 244–247° | (Dihydrochlorid) |

**Patentansprüche**

1. Neue Verbindungen der Formel I

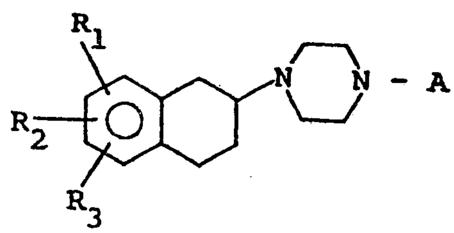

I

worin

$R_1$ Hydroxy, Alkoxy mit 1—4 C-Atomen, Alkanoyloxy mit 1—20 C-Atomen oder eine

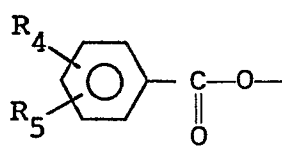

Gruppe,

$R_4$ und $R_5$ unabhängig voneinander Wasserstoff, Fluor, Chlor, Brom, Jod, Alkyl mit 1—4 C-Atomen oder Alkoxy mit 1—4 C-Atomen oder

$R_4$ und $R_5$ an benachbarten C-Atomen zusammen eine Methylendioxygruppe,

$R_2$ und $R_3$ unabhängig voneinander Wasserstoff, Hydroxy, Alkoxy mit 1—4 C-Atomen, Alkanoyloxy mit 1—20 C-Atomen oder eine

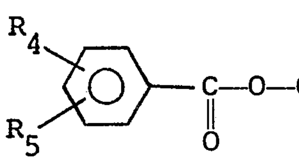

Gruppe oder

$R_1$ und $R_2$ an benachbarten C-Atomen zusammen eine Methylendioxygruppe,

A a) eine

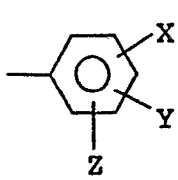

Gruppe,

X, Y und Z unabhängig voneinander Wasserstoff, Hydroxy, Alkyl mit 1—4 C-Atomen, Alkoxy mit 1—4 C-Atomen, Alkanoyloxy mit 1—20 C-Atomen,

eine 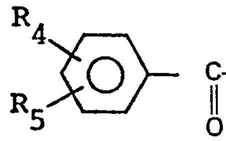 —D—Gruppe, Fluor, Chlor,

Brom, Jod, $CF_3$, SH, Alkylthio mit 1—4 C-Atomen oder Alkanoylthio mit 1—20 C-Atomen oder X und Y an benachbarten C-Atomen zusammen eine Methylendioxygruppe,

D O oder S

b) eine 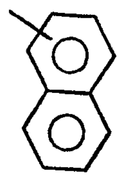 Gruppe,

c) einen Fünf- oder Sechsring der Formel

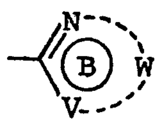

worin

V entweder zweiwertig ist und für O, S, NH oder $CH_2$ steht oder dreiwertig ist und für N oder CH steht,
W für eine gesättigte oder ungesättigte Alkylenkette mit 2 oder 3 C-Atomen steht und der Ring Ⓑ eine, zwei oder drei Doppelbindungen enthalten kann,
bedeuten sowie ihre Säureadditionssalze.

2. Verbindungen gemäss Anspruch 1, worin $R_1$ eine Hydroxygruppe bedeutet.
3. Verbindungen gemäss Anspruch 1 oder 2, worin $R_1$ sich in Stellung 6 befindet.
4. Verbindungen gemäss Anspruch 1, 2 oder 3, worin $R_2$ und $R_3$ Wasserstoff bedeuten.
5. Verbindungen gemäss einem der Ansprüche 1 bis 4, worin

A für eine 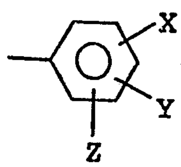 -Gruppe steht.

6. Verbindungen gemäss einem der Ansprüche 1 bis 5, worin X für Alkyl oder Alkoxy steht.

7. Verbindungen gemäss einem der Ansprüche 1 bis 6, worin X sich in Stellung 2 des Phenylrestes befindet.

8. 1,2,3,4-Tetrahydro-6-hydroxy-2-[4-(2-methylphenyl)piperazinyl-1]-naphthalin sowie seine Säureadditionssalze.

9. 1,2,3,4-Tetrahydro-6-hydroxy-2-[4-(2-methoxyphenyl)piperazinyl-1]-naphthalin sowie seine Säureadditionssalze.

10. Verbindungen gemäss Anspruch 1, worin
$R^1$ Hydroxy, Alkoxy mit 1—4 C-Atomen, Alkanyloxy mit 1—4 C-Atomen oder Benzyloxy,
$R_2$ und $R_3$ unabhängig voneinander Wasserstoff, Hydroxy, Alkoxy mit 1—4 C-Atomen, Alkanoyloxy mit 1—4 C-Atomen oder Benzoyloxy oder
$R_1$ und $R_2$ an benachbarten C-Atomen zusammen eine Methylendioxygruppe,

A eine 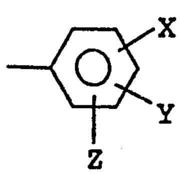 Gruppe,

X, Y und Z unabhängig voneinander Wasserstoff, Hydroxy, Alkyl mit 1—4 C-Atomen, Alkoxy mit 1—4 C-Atomen, Alkanoyloxy mit 1—4 C-Atomen, Benzyloxy, Fluor, Chlor oder $CF_3$ oder

X und Y an benachbarten C-Atomen zusammen eine Methylendioxygruppe bedeuten.

11. Verbindungen gemäss Anspruch 1, worin

$R^1$ Hydroxy, Alkoxy mit 1—4 C-Atomen, Alkanoyloxy mit 1—20 C-Atomen oder eine

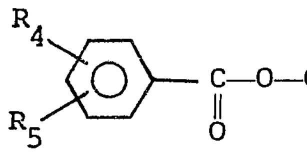
Gruppe,

$R_4$ und $R_5$ unabhängig voneinander Wasserstoff, Fluor, Chlor, Brom, Jod, Alkyl mit 1—4 C-Atomen oder Alkoxy mit 1—4 C-Atomen oder

$R_4$ und $R_5$ an benachbarten C-Atomen zusammen eine Methylendioxygruppe,

$R_2$ und $R_3$ unabhängig voneinander Wasserstoff, Hydroxy, Alkoxy mit 1—4 C-Atomen, Alkanoyloxy mit 1—20 C-Atomen oder eine

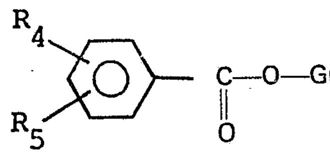
Gruppe oder

$R_1$ und $R_2$ an benachbarten C-Atomen zusammen eine Methylendioxygruppe,

A eine 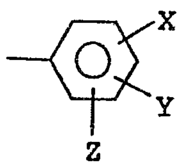 Gruppe,

X, Y und Z unabhängig voneinander Wasserstoff, Hydroxy, Alkyl mit 1—4 C-Atomen, Alkoxy mit 1—4 C-Atomen, Alkanoyloxy mit 1—20 C-Atomen, eine

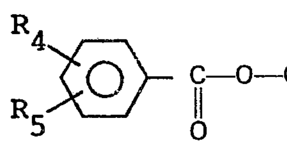
Gruppe, Fluor, Chlor, Brom, Jod,

$CF_3$, SH oder Alkylthio mit 1—4 C-Atomen oder

X und Y an benachbarten C-Atomen zusammen eine Methylendioxygruppe bedeuten.

12. Verfahren zur Herstellung der Verbindungen der Formel I und ihren Säureadditionssalzen gemäss Anspruch 1, dadurch gekennzeichnet, dass man

a) zur Herstellung der Verbindungen der Formel Ia

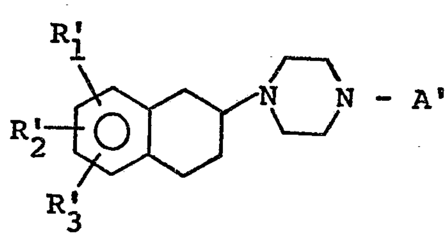
Ia

worin

$R_1'$ für eine Alkoxygruppe mit 1—4 C-Atomen und

$R_2'$ und $R_3'$ unabhängig voneinander für Wasserstoff oder eine Alkoxygruppe mit 1—4 C-Atomen oder

$R_1'$ und $R_2'$ zusammen für eine —O—$CH_2$—O—Gruppe stehen und

A' die gleichen Bedeutungen wie A, mit Ausnahme des durch mindestens eine Alkanoyloxy-,

Alkanoyl- oder 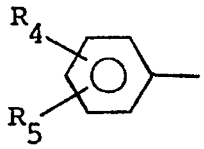 COD—Gruppe

substituierten Phenylrestes, hat Verbindungen der Formel II

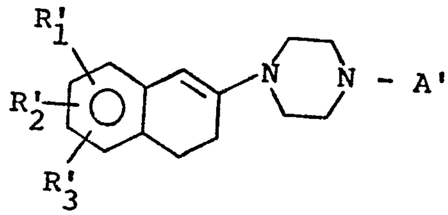 II

reduziert,

b) zur Herstellung der Verbindungen der Formel Ib

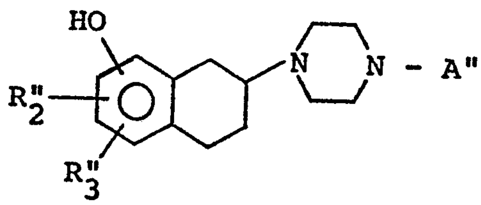 Ib

worin

$R_2''$ und $R_3''$ unabhängig voneinander für Wasserstoff oder Hydroxy und

A″ für eine 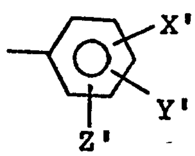 Gruppe,

worin

X′, Y′ und Z′ unabhängig voneinander Wasserstoff, Hydroxy, Alkyl mit 1—4 C-Atomen, Fluor, Chlor, Brom, Jod, $CF_3$ oder SH bedeuten,

oder für eine der oben unter b) und c) angegebenen Bedeutungen für A stehen,

Verbindungen der Formel Ia einer Aetherspaltung unterwirft,

c) zur Herstellung der Verbindungen der Formel Ic

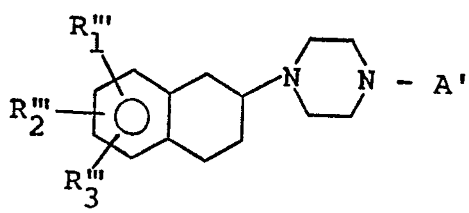 Ic

worin

$R_1'''$ für Hydroxy oder Alkoxy mit 1—4 C-Atomen,

$R_2'''$ und $R_3'''$ unabhängig voneinander für Wasserstoff, Hydroxy oder Alkoxy mit 1—4 C-Atomen stehen,

Verbindungen der Formel III

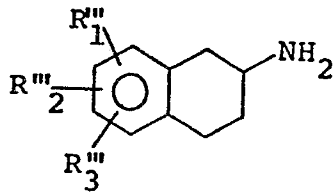

III

mit Verbindungen der Formel IV

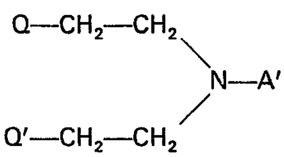

IV

worin Q und Q' für den Säurerest eines reaktiven Esters stehen, kondensiert,

d) zur Herstellung der Verbindungen der Formel I, worin mindestens einer der Substituenten $R_1$, $R_2$, $R_3$, X, Y oder Z für eine Alkanoyloxy- oder

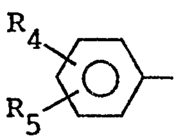

COO—Gruppe und/oder einer der Substituenten X, Y oder Z für eine Alkanoylthio- oder

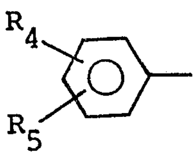

COS—Gruppe steht,

Verbindungen der Formel I, worin mindestens einer der Substituenten $R_1$, $R_2$, $R_3$, X, Y oder Z für eine freie OH-Gruppe und/oder einer der Substituenten X, Y oder Z für eine freie SH-Gruppe steht, mit einem reaktiven Derivat einer Alkancarbonsäure mit 1—20 C-Atomen oder einer aromatischen Carbonsäure der Formel

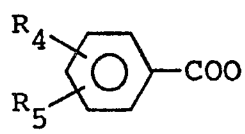

acyliert

und die erhaltenen Verbindungen der Formel I gegebenenfalls in ihre Säureadditionssalze überfuhrt.

13. Heilmittel enthaltend mindestens eine Verbindung gemäss Anspruch 1.

**Revendications**

1. Nouveaux composés de formula I

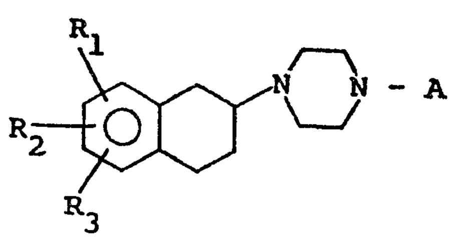

(I)

dans laquelle

$R_1$ signifie un groupe hydroxy, alcoxy contenant de 1 à 4 atomes de carbone, alcanoyloxy contenant de 1 à 20 atomes de carbone ou un groupe

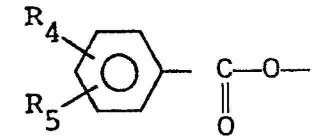

où $R_4$ et $R_5$ signifient, indépendamment l'un de l'autre, l'hydrogène, le fluor, le chlore, le brome, l'iode, un groupe alkyle contenant de 1 à 4 atomes de carbone ou alcoxy contenant de 1 à 4 atomes de carbone, ou bien

$R_4$ et $R_5$ sont situés sur des atomes de carbone adjacents et forment ensemble un groupe méthylenedioxy,

$R_2$ et $R_3$ signifient, indépendamment l'un de l'autre, l'hydrogène, un groupe hydroxy, alcoxy contenant de 1 à 4 atomes de carbone, alcanoyloxy contenant de 1 à 20 atomes de carbone, ou un groupe

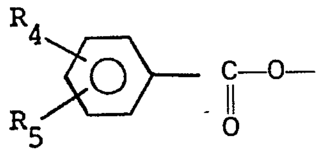

ou bien

$R_1$ et $R_2$ sont situés sur des atomes de carbone adjacents et forment ensemble un groupe méthylènedioxy,

A signifie

a) un groupe 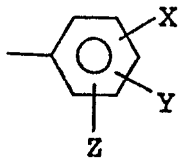

où, X, Y et Z signifient, indépendamment les uns des autres, l'hydrogène, un groupe hydroxy, alkyle contenant de 1 à 4 atomes de carbone, alcoxy contenant de 1 à 4 atomes de carbone, alcanoyloxy contenant de 1 à 20 atomes de carbone, un groupe

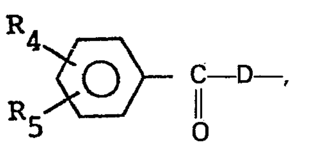

le fluor, le chlore, le brome, l'iode, $CF_3$, SH, un groupe alkylthio contenant de 1 à 4 atomes de carbone ou alcanoylthio contenant de 1 à 20 atomes de carbone, ou

X et Y sont situés sur des atomes de carbone adjacents et forment ensemble un groupe méthylènedioxy,

D signifie O ou S,

b) un groupe

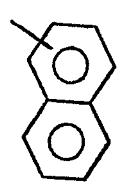

c) un cycle à 5 ou 6 chaînons de formule

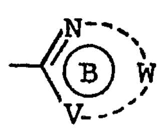

où V est soit bivalent et représente O, S, NH ou $CH_2$, soit trivalent et représente N ou CH, W est une chaîne alkylène saturée ou insaturée contenant 2 ou 3 atomes de carbone, et le cycle ® peut contenir 1, 2 ou 3 doubles liaisons,

ainsi que leurs sels d'addition d'acides.

2. Composés selon la revendication 1, dans lesquels $R_1$ signifie un groupe hydroxy.

3. Composés selon la revendication 1 ou 2, dans lesquels $R_1$ se trouve en position 6.

4. Composés selon la revendication 1, 2 ou 3, dans lesquels $R_2$ et $R_3$ signifient l'hydrogène.

5. Composés selon l'une des revendications 1 à 4, dans lesquels A signifie un groupe

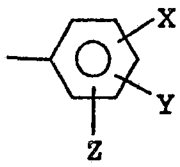

6. Composés selon l'une des revendications 1 à 5, dans lesquels X représente un groupe alkyle ou alcoxy.

7. Composés selon l'une des revendications 1 à 6, dans lesquels X se trouve en position 2 du reste phényle.

8. Le 1,2,3,4-tétrahydro-6-hydroxy-2-[4-(2-méthylphényl)piperazinyl-1]naphtalène ainsi que ses sels d'addition d'acides.

9. Le 1,2,3,4-tétrahydro-6-hydroxy-2-[4-(2-méthoxyphényl)pipérazinyl-1]naphtalène ainsi que ses sels d'addition d'acides.

10. Composés selon la revendication 1, dans lesquels

$R_1$ signifie un groupe hydroxy, alcoxy contenant de 1 à 4 atomes de carbone, alcanoyloxy contenant de 1 à 4 atomes de carbone ou benzoyloxy,

$R_2$ et $R_3$ signifient, indépendamment l'un de l'autre, l'hydrogène, un groupe hydroxy, alcoxy contenant de 1 à 4 atomes de carbone, alcanoyloxy contenant de 1 à 4 atomes de carbone ou benzoyloxy, ou bien

$R_1$ et $R_2$ sont situés sur des atomes de carbone adjacents et forment ensemble un groupe méthylènedioxy,

A signifie un groupe

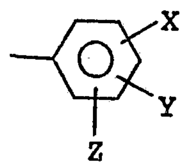

où, X, Y et Z signifient, indépendamment les uns des autres, l'hydrogène, un groupe hydroxy, alkyle contenant de 1 à 4 atomes de carbone, alcoxy, contenant de 1 à 4 atomes de carbone, alcanoyloxy contenant de 1 à 4 atomes de carbone, benzoyloxy, le fluor, le chlore ou $CF_3$, ou bien X et Y sont situés sur des atomes de carbone adjacents et forment ensemble un groupe méthylènedioxy.

11. Composés selon la revendication 1, dans lesquels $R_1$ signifie un groupe hydroxy, alcoxy contenant de 1 à 4 atomes de carbone, alcanoyloxy contenant de 1 à 20 atomes de carbone ou un groupe

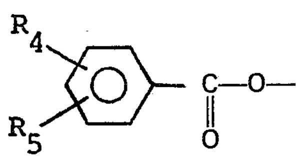

où $R_4$ et $R_5$ signifient, indépendamment l'un de l'autre, l'hydrogène, le fluor, le chlore, le brome, l'iode, un groupe alkyle contenant de 1 à 4 atomes de carbone ou alcoxy contennant de 1 à 4 atomes de carbone, ou bien

$R_4$ et $R_5$ sont situés sur des atomes de carbone adjacents et forment ensemble un groupe méthylènedioxy,

$R_2$ et $R_3$ signifient, indépendamment l'un de l'autre, l'hydrogène, un groupe hydroxy, alcoxy contenant de 1 à 4 atomes de carbone, alcanoyloxy contenant de 1 à 20 atomes de carbone ou un groupe

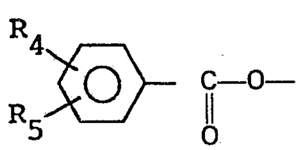

ou bien
$R_1$ et $R_2$ sont situés sur des atomes de carbone adjacents et forment ensemble un groupe méthylènedioxy,
A signifie un groupe

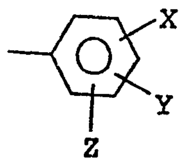

où X, Y et Z signifient, indépendamment les uns des autres, l'hydrogène, un groupe hydroxy, alkyle contennant de 1 à 4 atomes de carbone, alcoxy contenant de 1 à 4 atomes de carbone, alcanoyloxy contenant de 1 à 20 atomes de carbone, un groupe

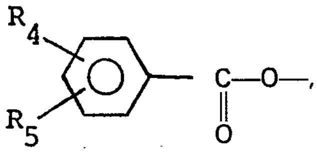

le fluor, le chlore, le brome, l'iode, $CF_3$, SH ou un groupe alkylthio contenant de 1 à 4 atomes de carbone, ou bien
X et Y sont situés sur des atomes de carbone adjacents et forment ensemble un groupe méthylènedioxy.

12. Procédé pour la préparation des composés de formule I et de leurs sels d'addition d'acides selon la revendication 1, caractérisé en ce que
a) pour préparer les composés de formule Ia

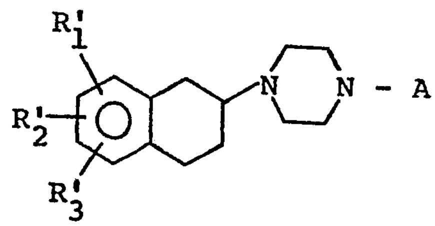

(Ia)

dans laquelle $R_1'$ représente un groupe alcoxy contenant de 1 à 4 atomes de carbone,
$R_2'$ et $R_3'$ représentent, indépendamment l'un de l'autre, l'hydrogène ou un groupe alcoxy contenant de 1 à 4 atomes de carbone, ou bien
$R_1'$ et $R_2'$ forment ensemble un groupe $-O-CH_2-O-$ et A' a les mêmes significations que A, à l'exception du reste phényle substitué par au moins un groupe alcanoyloxy, alcanoylthio ou

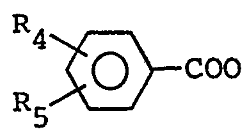

on réduit des composés de formule II

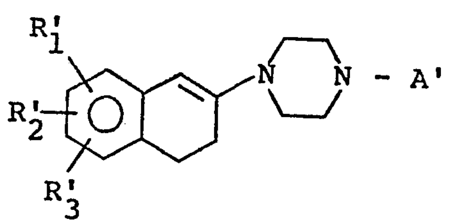

(II)

b) pour préparer les composés de formule Ib

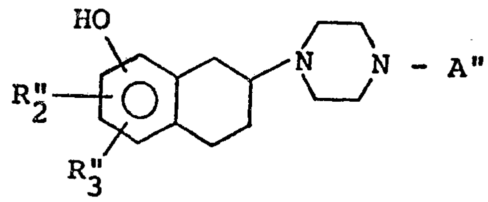

(Ib)

dans laquelle $R_2''$ et $R_3''$ représentent, indépendamment l'un de l'autre, l'hydrogène ou un groupe hydroxy, et A″ représente un groupe

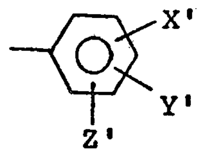

où X′, Y′ et Z′ représentent, indépendamment les uns des autres, l'hydrogène, un groupe hydroxy, alkyle contenant de 1 à 4 atomes de carbone, le fluor, le chlore, le brome, l'iode, $CF_3$ ou SH, ou bien possède l'une des significations données pour A sous b) et c),
on scinde le groupe éther des composés de formule Ia,
c) pour préparer les composés de formule Ic

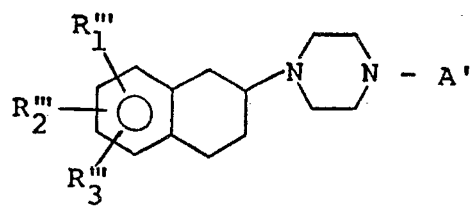

(Ic)

dans laquelle $R_1'''$ représente un groupe hydroxy ou alcoxy contenant de 1 à 4 atomes de carbone, $R_2'''$ et $R_3'''$ représentent, indépendamment l'un de l'autre, l'hydrogène, un groupe hydroxy ou alcoxy contenant de 1 à 4 atomes de carbone,
on condense des composés de formule III

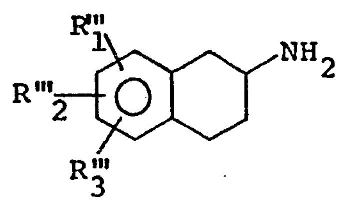

(III)

avec des composés de formule IV

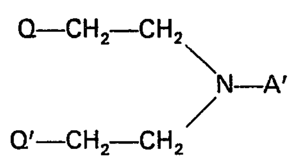

dans laquelle Q et Q′ représentent le reste acide d'un ester réactif,
d) pour préparer les composés de formule I où l'un au moins des substituants $R_1$, $R_2$, $R_3$, X, Y ou Z représente un groupe alcanoyloxy ou

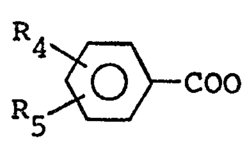

et/ou l'un des substituants X, Y ou Z représente un groupe alcanoylthio ou

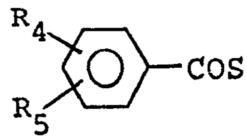

on acyle les composés de formule I où l'un au moins des substituants $R_1$, $R_2$, $R_3$, X, Y ou Z représente un groupe hydroxy libre et/ou l'un des substituants X, Y ou Z représente un groupe SH libre, avec un dérivé réactif d'un acide alcanoïque contenant de 1 à 20 atomes de carbone ou d'un acide carboxylique aromatique de formule

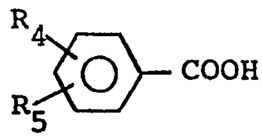

et on transforme éventuellement les composés de formule I ainsi obtenus en leurs sels d'addition d'acides.

13. Un médicament contenant au moins un composé selon la revendication 1.

**Claims**

1. New compounds of formula I,

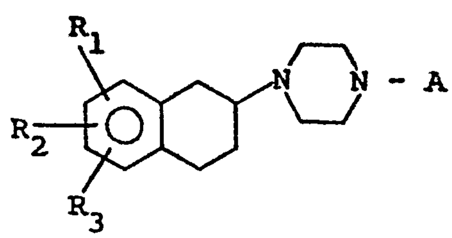

I

wherein $R_1$ is hydroxy, alkoxy of 1 to 4 carbon atoms, alkanoyloxy of 1 to 20 carbon atoms or a

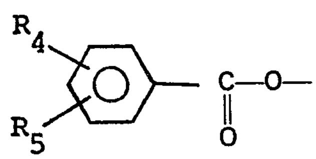 group,

wherein

$R_4$ and $R_5$ are, independently, hydrogen, fluorine, chlorine, bromine, iodine, alkyl of 1 to 4 carbon atoms or alkoxy of 1 to 4 carbon atoms, or

$R_4$ and $R_5$ together are a methylenedioxy group and are bonded to adjacent carbon atoms,

$R_2$ and $R_3$ are, independently, hydrogen, hydroxy, alkoxy of 1 to 4 carbon atoms, alkanoyloxy of 1 to 20 carbon atoms or a

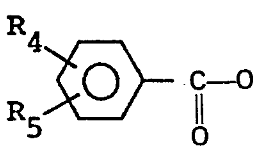 group, or

$R_1$ and $R_2$ together are a methylenedioxy group and are bonded to adjacent carbon atoms,

A is

a) a 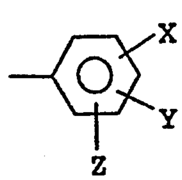 group,

wherein

X, Y and Z are, independently, hydrogen, hydroxy, alkyl of 1 to 4 carbon atoms, alkoxy of 1 to 4 carbon atoms, alkanoyloxy of 1 to 20 carbon atoms,

a 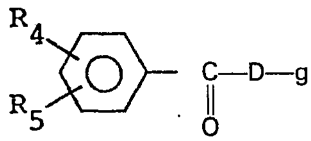 group, fluorine

chlorine, bromine, iodine, $CF_3$, SH, alkylthio of 1 to 4 carbon atoms or alkanoylthio of 1 to 20 carbon atoms, or

X and Y together are methylenedioxy and are bonded to adjacent carbon atoms,

D is O or S

b) a 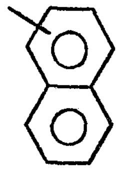 group,

c) a five or six membered ring of formula

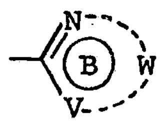

wherein

V is either divalent and signifies O, S, NH or $CH_2$ or trivalent and signifies N or CH,

W is a saturated or unsaturated alkylene chain of 2 or 3 carbon atoms

and ring B can contain 1, 2 or 3 double bonds, and their acid addition salts.

2. Compounds according to claim 1 wherein $R_1$ is a hydroxyl group.

3. Compounds according to claim 1 or 2 wherein $R_1$ is located at position 6.

4. Compounds according to claim 1, 2 or 3 wherein $R_2$ and $R_3$ are hydrogen.

5. Compounds according to any one of claims 1 to 4 wherein A signifies a group

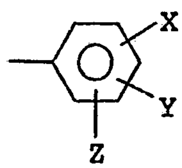

6. Compounds according to any one of claims 1 to 5 wherein X is alkyl or alkoxy.

7. Compounds according to any one of claims 1 to 6 wherein X is in position 2 of the phenyl radical.

8. 1,2,3,4-tetrahydro-6-hydroxy-2-[4-(2-methylphenyl)piperazinyl-1]naphthalene and its acid addition salts.

9. 1,2,3,4-tetrahydro-6-hydroxy-2-[4-(2-methoxyphenyl)piperazinyl-1]naphthalene and its acid addition salts.

10. Compounds of claim 1 wherein $R_1$ is hydroxy, alkoxy of 1 to 4 carbon atoms, alkanoyloxy of 1 to 4 carbon atoms or benzoyloxy, $R_2$ and $R_3$ are, independently, hydrogen, hydroxy, alkoxy of 1 to 4 carbon atoms, alkanoyloxy of 1 to 4 carbon atoms or benzoyloxy, or $R_1$ and $R_2$ are together a methylene-dioxy group and are bonded to adjacent carbon atoms, A is a

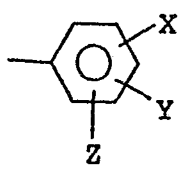 group wherein X, Y and Z

are, independently, hydrogen, hydroxy, alkyl of 1 to 4 carbon atoms, alkoxy of 1 to 4 carbon atoms, alkanoyloxy of 1 to 4 carbon atoms, benzoyloxy, fluorine, chlorine or $CF_3$, or X and Y are together a methylenedioxy group and are bonded to adjacent carbon atoms.

11. Compounds of claim 1 wherein $R_1$ is hydroxy, alkoxy of 1 to 4 carbon atoms, alkanoyloxy of 1 to 20 carbon atoms or a

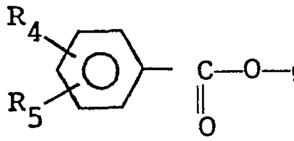
group,

wherein $R_4$ and $R_5$ are, independently, hydrogen, fluorine, chlorine, bromine, iodine, alkyl of 1 to 4 carbon atoms or alkoxy of 1 to 4 carbon atoms, or $R_4$ and $R_5$ are together a methylendioxy group and are bonded to adjacent carbon atoms, $R_2$ and $R_3$ are, independently, hydrogen, hydroxy, alkoxy of 1 to 4 carbon atoms, alkanoyloxy of 1 to 20 carbon atoms or a

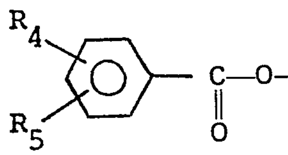
group, or

$R_1$ and $R_2$ together are a methylenedioxy group and are bonded to adjacent carbon atoms.

A is

a 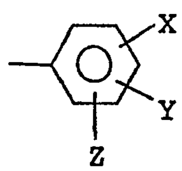 group,

wherein

X, Y and Z are, independently, hydrogen, hydroxy, alkyl of 1 to 4 carbon atoms, alkoxy of 1 to 4 carbon atoms, alkanoyloxy of 1 to 20 carbon atoms,

a 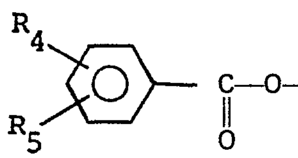 group, fluorine

chlorine, bromine, iodine, $CF_3$, SH, alkylthio of 1 to 4 carbon atoms or

X and Y together are a methylenedioxy group and are bonded to adjacent carbon atoms.

12. Process for the production of compounds of formula I and their acid addition salts according to claim 1, characterised by

a) producing compounds of formula Ia,

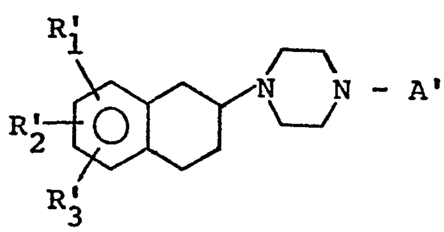
Ia

wherein

$R_1'$ is alkoxy of 1 to 4 carbon atoms,

$R_2'$ and $R_3'$ are, independently, hydrogen or alkoxy of 1 to 4 carbon atoms, or

$R_1'$ and $R_2'$, together are a $—O—CH_2—O—$ group and,

A' has the same significances as A other than a phenyl residue substituted by at least one of the groups alkanoyloxy, alkanoylthio or

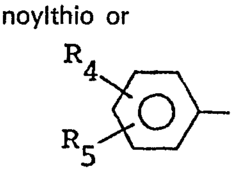
COD—,

by reducing compounds of formula II,

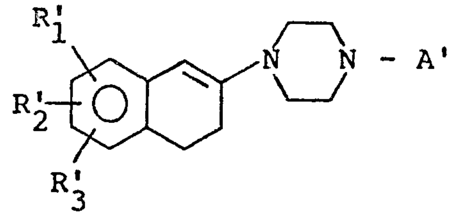

II

b) producing compounds of formula Ib,

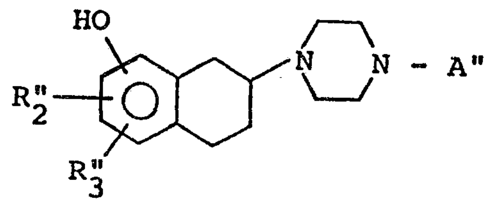

Ib

wherein each of $R_2''$ and $R_3''$ is, independently, hydrogen or hydroxy and

A″ is a 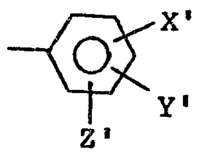 group,

wherein each of X′, Y′ and Z′ is, independently, hydrogen, hydroxy, alkyl of 1 to 4 carbon atoms, fluorine, chlorine, bromine, iodine, $CF_3$ or SH or a moiety as previously defined for A under b) and c) above, by subjecting compounds of formula Ia to an ether cleavage reaction,
c) producing compounds of formula Ic,

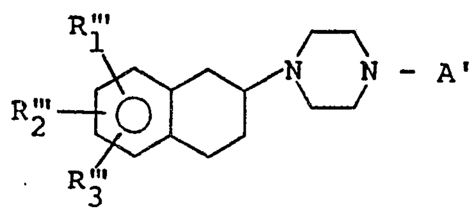

Ic

wherein $R_1'''$ is hydroxy or alkoxy of 1 to 4 carbon atoms, and
each of $R_2'''$ and $R_3'''$ is, independently, hydrogen, hydroxy or alkoxy of 1 to 4 carbon atoms, by condensing compounds of formula III,

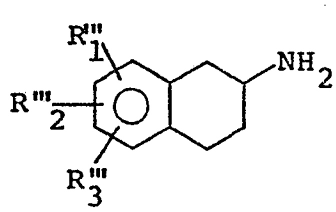

III

with compounds of formula IV,

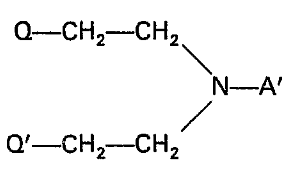

IV

wherein Q and Q′ signify an acid residue of a reactive ester or

d) producing compounds of formula I wherein at least one of the substituents $R_1$, $R_2$, $R_3$, X, Y and Z is an alkanoyloxy or a

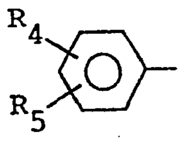

COO—group,

and/or one of the substituents X, Y and Z is an alkanoylthio or

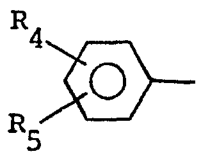

COS group,

by acylating compounds of formula I, wherein at least one of the substituents $R_1$, $R_2$, $R_3$, X, Y and Z is a free hydroxy group and/or one of the substituents X, Y and Z is a free SH group, with a reactive derivative of an alkanoic acid of 1 to 20 carbon atoms or an aromatic carboxylic acid of formula

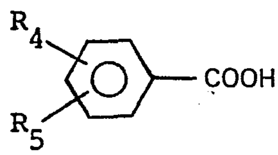

and the resulting compounds of formula I are optionally converted into their acid addition salts.

13. Therapeutic compositions containing at least one compound according to claim 1.